# FASCICULE DE BREVET EUROPEEN

(11) **EP 1 675 557 B1**
(45) Date de publication et mention de la délivrance du brevet: **23.04.2008**
(21) Numéro de dépôt: 04805286.4
(22) Date de dépôt: 22.10.2004
(51) Int. Cl.: A61K 8/18, A61K 31/557, A61K 36/18

(54) **COMPOSITION COMPRENANT UNE LACTONE SESQUITERPENIQUE**
EIN SESQUITERPEN-LACTON ENTHALTENDE ZUSAMMENSETZUNG
COMPOSITION COMPRISING A SESQUITERPENE LACTONE

(30) Priorité: 22.10.2003 FR 0312348
(43) Date de publication de la demande: 05.07.2006
(73) Titulaire: Greenpharma, 63360 Saint Beauzire (FR)
(72) Inventeur: BERNARD, Philippe, F-18800 Farges-en-Septaine (FR)
(74) Mandataire: Breese, Pierre
(86) Numéro de dépôt international: PCT/FR2004/002724
(87) Numéro de publication internationale: WO 2005/039523

(56) Documents cités:
- WO-A-01/12206
- WO-A-01/60326
- WO-A-98/55075
- FR-A- 2 807 319
- US-A- 5 663 196
- US-A- 5 905 089
- DATABASE CAPLUS [Online] CHEMICAL ABSTRACTS SERVICE, COLUMBUS, OHIO, US; ZAFRA-POLO ET AL.: "antiinflammatoty activity of sesquiterpene lactones from Artemisia barrelieri in rats" XP002229210 extrait de STN Database accession no. 1991:441302
- DATABASE CAPLUS [Online] CHEMICAL ABSTRACTS SERVICE, COLUMBUS, OHIO, US; RIDRIGUEZ ET AL.: "preliminary study of the effects produced in th lipid fraction of several organs of the rat after administration of vulgarin" XP002229211 extrait de STN Database accession no. 1976:571817 cité dans la demande
- DATABASE CAPLUS [Online] CHEMICAL ABSTRACTS SERVICE, COLUMBUS, OHIO, US; LEE ET AL.: "a new guaianolide as apoptosis inhibitor from Chrysanthemum boreale" XP002229212 extrait de STN Database accession no. 2001:656390
- DATABASE WPI Week 199746 Derwent Publications Ltd., London, GB; AN 1997-497264 XP002229218 & JP 09 234020 A (NIPPON SHINYAKU CO) 9 septembre 1997 (1997-09-09)
- DATABASE CAPLUS [Online] CHEMICAL ABSTRACTS SERVICE, COLUMBUS, OHIO, US; ZHENG ET AL.: "a new antimicrobial sesquiterpene lactone from Artemisia giraldii" XP002229213 extrait de STN Database accession no. 1996:747384
- DATABASE CAPLUS [Online] CHEMICAL ABSTRACTS SERVICE, COLUMBUS, OHIO, US; GIORDANO ET AL.: "the gastric cytoprotective effect of several sesquiterpene lactones" XP002229214 extrait de STN Database accession no. 1990:584222
- DATABASE CAPLUS [Online] CHEMICAL ABSTRACTS SERVICE, COLUMBUS, OHIO, US; XP002229215 extrait de STN Database accession no. 1994:200171 & JP 05 306231 A (POLA KASEI KOGYO KK) 19 novembre 1993 (1993-11-19)
- DATABASE CAPLUS [Online] CHEMICAL ABSTRACTS SERVICE, COLUMBUS, OHIO, US; XP002229216 extrait de STN Database accession no. 1998:724152 & JP 10 298092 A (NOEVIR CO) 10 novembre 1998 (1998-11-10)
- DATABASE CAPLUS [Online] CHEMICAL ABSTRACTS SERVICE, COLUMBUS, OHIO, US; GONZALEZ ET AL.: "cytostatic activity of sesquiterpene lactones from Compositae of the Canary Islands" XP002229217 extrait de STN Database accession no. 1978:608918
- DATABASE CAPLUS [Online] XP002277532 extrait de STN Database accession no. 2002:817848
- DATABASE WPI Section Ch, Week 200231 Derwent Publications Ltd., London, GB; Class B04, AN 2002-265097 XP002277533 & KR 2001 100 218 A (DINAMED CO LTD) 14 novembre 2001 (2001-11-14)

## Description

La présente invention concerne des compositions comprenant à titre d'agent actif au moins une lactone sesquiterpénique choisie parmi la vulgarine, la 4-épivulgarine, la 2,3 dihydrovulgarine ainsi que leurs sels et un ou plusieurs véhicules ou excipients adaptés à une application topique, ou au moins un extrait végétal contenant de telle lactone sesquiterpénique. L'invention concerne aussi l'utilisation d'une telle composition dans les domaines pharmaceutiques, dermatologiques ou cosmétiques, pour traiter et/ou prévenir des troubles esthétiques résultant d'un processus de mort cellulaire par apoptose. L'invention concerne également le domaine nutraceutique.

Les lactones sesquiterpéniques sont présentes dans des végétaux, parmi lesquels on peut citer plus particulièrement les Astéracées (ou composées), les Lauracées, les Frullaniacées et les Magnoliacées. On les trouve donc dans tous les produits qui en sont dérivés.

Il s'agit aussi de dérivés biosynthétiques de germacranolide de formule (I) et principalement d'eudesmanolides et de guaianolides.

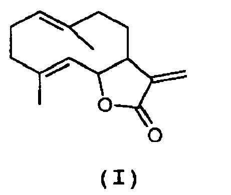

Cette structure générale est à l'origine de nombreux dérivés notamment issus d'importants réarrangements structuraux.

Parmi les lactones sesquiterpéniques, l'invention envisage tout particulièrement la vulgarine de formule (II) ci-dessous.

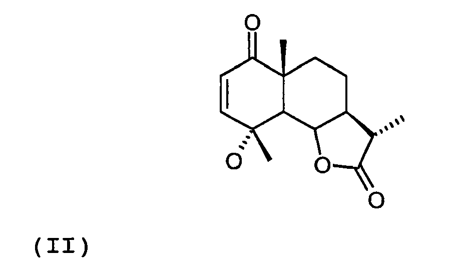

La vulgarine est une lactone sesquiterpénique du groupe des eudesmanolides décrite pour la première fois en 1961 par Rybalko (Rybalko K.S., Collect. Czech. Chem. Commun., 1961, 26, 2909). Il est connu que cette molécule possède des propriétés hypoglycémiantes (Rodriguez de Vera C., Boll. Chim. Farm., 1976, 115, 445), antitumorale (Jeffery B. Harborne and H. Baxter, eds. 1983. Phytochemical Dictionary. A Handbook of Bioactive Compounds from Plants. Taylor & Frost, London. pp 791) et antibactérienne (Pickman A.K., Bioch. Syst. Ecol., 1986, 14, 255).

La vulgarine ainsi que ses dérivés lactones sesquiterpéniques sont essentiellement extraits des plantes de la famille des Astéracées, notamment du genre *Artemisia* et *Achillea.* La littérature fait état de nombreuses espèces du genre *Artemisia,* contenant de la vulgarine et ses dérivés. Un article récent indique ces références (Mansilla H. and Palenzuela J.A., Phytochemistry, 1999, 51, 995). Il en est de même pour les plantes du genre *Achillea* (Glasl S. et al, Phytochemistry, 1995, 38, 159 ; Kubelka W. et al, Bioch. Syst. Ecol., 1999, 27, 437).

*Artemisia* Sp et *Achillea* Sp sont des Astéracées largement répandues avec de nombreuses espèces. Elles ont déjà largement été décrites dans la littérature pour diverses propriétés, notamment antibactérienne, antipyrétique, anthelmintique, emménagogue... (Bruneton J., Pharmacognosie, Phytochimie-Plantes médicinales, deuxième édition Technique et Documentation-Lavoisier, 1993). Ces plantes sont décrites pour développer des lactones sesquiterpéniques, notamment des dérivés de la vulgarine.

On connaît dans l'art antérieur l'activité anti-inflammatoire de lactones sesquiterpèniques, notamment des dérivés de la vulgarine extraite de plantes de la famille des Artemisia (Chemical Abstracts Service, Columbus, Ohio, US ; Zafra-Polo et al. : « antiinflammatory activity of sesquiterpenes lactones from Artemisia barrelieri in rats » XP002229210 extrait STN Database accession no. 1991 :441302). On connaît aussi les effets de la vulgarine extraite de plantes du genre Artemisia comme agent hypoglycémique oral (Chemical Abstracts Service, Columbus, Ohio, US ; Ridriguez et al. : « preliminary study of the effects produced in the lipid fraction of several organs of the rat after administration of vulgarine » XP002229211 extrait STN Database accession no. 1976 :517817). On a encore rapporté l'utilisation d'un quaianolide comme inhibiteur de l'apoptose et de caspase (Chemical Abstracts Service, Columbus, Ohio, US ; Lee et al. « a new guaianolide as apoptosis inhibitor from Chrysanthemum boreale » XP002229212 extrait STU Database accession no. 2001 :656390).

Les travaux de recherche réalisés dans le cadre de la présente invention ont maintenant mis en évidence, de façon surprenante, que les lactones sesquiterpéniques, plus particulièrement la vulgarine, et des extraits végétaux les contenant, agissaient, de manière dose dépendante, comme inhibiteurs de caspases et inhibaient également la production d'interleukines et l'activité des macrophages.

Les caspases sont des enzymes appartenant à la famille des cysteinyl aspartarte protéases, essentiellement impliquées dans les processus de mort cellulaire par apoptose. Ce processus a été largement étudié ces dernières années, car il est à l'origine de nombreux intérêts thérapeutiques (Lockshin R.A. et al, Eds, When cells die, Wiley-Liss, New York, 1998). Actuellement, on dénombre 11 caspases humaines, subdivisées en 3 sous groupes selon leur homologie de séquence et la spécificité des substrats. Les caspases du groupe I, caspases 1, 4, 5 et 13, sont impliquées dans les processus inflammatoires, liés à la maturation de multiples cytokines pro-inflammatoires. Les caspases du groupe II, caspases 2, 3 et 7, sont impliquées dans le clivage de substrats apoptotiques. Enfin, le groupe III remembre les caspases 6, 8 et 9, impliquées dans l'activation des caspases du groupe II. Il semble que certaines caspases puissent avoir des fonctions redondantes étant donné la forte homologie des substrats clivés (Nicholson D.W. and Thornberry N.A., Trends Biochem. Sci., 1997, 22, 299). Toutefois, certains inhibiteurs sélectifs commencent à être mis en évidence (Lee D. et al, J. Med. Chem., 2001, 44, 2015). Les travaux de cristallographie ont permis d'accéder à la connaissance structurale de certaines caspases, ce qui a facilité la recherche de zones structurales permettant la sélectivité (Mittl P.R.E. et al, J. Biol. Chem., 1997, 272, 6539 ; Rotonda J., Nat. Struct. Biol., 1996, 3, 619).

Les caspases sont exprimées dans le cytosol de nombreux type cellulaire, y compris les cellules de la peau comme les mélanocytes, les kératinocytes, etc... ainsi que dans les cellules du cartilage comme les chondrocytes. Elles apparaissent également dans les cellules du tissus cérébral, où elles deviennent la cible de maladies neurodégénératives (Lee D., « Apoptotic caspase inhibitors : Progress towards identifying therapeutic agents », communication oral). Un article récent décrit que la caspase 3 est impliquée dans les processus de clivage d'un facteur de fragmentation de l'ADN qui induit la destruction de l'ADN (Boulares A.H. et al, J. Biol. Chem., 2001, sous presse). Un autre article scientifique mentionne que l'identification de ligands bloquant ces cibles auraient des vertus protectrices des mélanocytes et des fibroblastes (Zhang X.D. et al, FEBS Lett., 2000, 482, 193). Par ailleurs, il a été montré que de tels ligands favoriseraient la protection des réseaux de collagène et empêcheraient la mort cellulaire des cellules du cartilage (Lee D. et al, J. Biol. Chem., 2000, 275, 16007). Enfin, des propriétés anti-inflammatoires sont revendiquées étant donné l'implication de certaines caspases dans les processus de libération de cytokines pro-inflammatoires (Nicholson D.W. and Thornberry N.A., Trends Biochem. Sci., 1997, 22, 299). Les actions combinées anti-inflammatoire et de protection cellulaire et moléculaire d'inhibiteurs de caspases permettent de suggérer des applications thérapeutiques dans les troubles articulaires (Lee D. et al, J. Biol. Chem., 2000, 275, 16007).

La présente invention vise précisément à offrir de nouvelles compositions dermatologiques ou cosmétiques capables d'inhiber les caspases et notamment la caspase 3, et donc tout particulièrement utile pour le traitement ou la prévention de pathologies ou de troubles esthétiques résultant d'un processus de mort cellulaire par apoptose. Ce but est atteint grâce à des compositions dermatologiques ou cosmétiques comprenant comme agent actif au moins une lactone sesquiterpénique choisie parmi la vulgarine, la 4-épivulgarine, la 2,3 dihydrovulgarine ainsi que leurs sels et un ou plusieurs véhicules ou excipients adaptés à une application topique. L'invention concerne aussi une composition comprenant au moins un extrait végétal contenant de la vulgarine et/ou de la 4-épivulgarine, et/ou de la 2,3 dihydrovulgarine. L'invention envisage tout particulièrement comme agent actif dans les compositions ci-dessus la vulgarine ou un extrait végétale la contenant.

Il peut aussi s'agir de sels ou complexes acceptables. Des exemples de tels sels comprennent, mais ne se limitent pas à, des sels formés avec des acides inorganiques, comme ceux choisis dans le groupe comprenant les acides chlorhydrique, bromhydrique, sulfurique, phosphorique, nitrique ou équivalent, ou des sels formés avec des acides organiques tels que les acides acétique, oxalique, tartrique, succinique, malique, fumarique, maléique, ascorbique, benzoique, tannique, alginique, polyglutamique, naphtalène sulfonique, naphtalène disulfonique et polygalacturonique.

On entend tout particulièrement par « extrait végétal » un extrait d'*Artemisia* Sp ou d'*Achillea* Sp. Il s'agit plus spécialement d'un extrait de cellules d'*Artemisia* Sp ou d'*Achillea* Sp et tout spécifiquement d'un extrait de cellules d'au moins un végétal du genre *Artemisia* Sp ou *Achillea* Sp de la famille des Astéracées. Ce matériel cellulaire peut être obtenu par culture *in vitro* ou *in vivo.* Par culture *in vitro,* on entend l'ensemble des techniques connues de l'homme du métier qui permettent de manière artificielle l'obtention d'un végétal ou d'une partie d'un végétal. Par culture *in vivo,* on entend l'ensemble des techniques de cultures qui permettent d'obtenir un végétal ou une partie d'un végétal. Ainsi, l'extrait peut être un extrait d'organe (racine, tige, feuille, écorce), voire de cellules d'organe, d'au moins une plante du genre *Artemisia* Sp ou *Achillea* Sp de la famille des Astéracées, ou encore un extrait de cellules indifférenciées d'au moins une telle plante. Ces extraits sont enrichis dans des proportions variables selon le type d'extrait, en lactones sesquiterpéniques. Ces extraits purifiés présentent ainsi l'avantage d'être affranchis de tout problème de toxicité par rapport à l'extrait brut. Plus particulièrement, trois formules d'extraction / purification peuvent être envisagées : (i) un extrait total de la plante, (ii) un extrait visant à concentrer les lactones sesquiterpéniques et enfin, (iii) l'obtention de lactones sesquiterpéniques pures.

Toute méthode d'extraction ou de purification connue de l'homme du métier peut être utilisée selon l'invention. On peut en particulier citer les extraits alcooliques (notamment méthanoliques, éthanoliques), aqueux ou des extraits utilisant des solvants tels que les cétones, les esters, les éthers, les polyols, les solvants chlorés et les mélanges d'au moins deux des solvants précités, comme les extraits hydroalcooliques.

A titre d'exemple de préparation d'extraits d'*Artemisia* Sp ou d'*Achillea* Sp et de lactones sesquiterpéniques purifiées selon l'invention, on peut citer les protocoles suivants :
- Extrait total : Dans une première étape, on broie le matériel végétal frais, congelé ou sec dans une solution aqueuse à froid, dans une seconde étape, on filtre la solution aqueuse qui est finalement stérilisée. Cette solution aqueuse correspond à l'extrait. Cet extrait peut ensuite être séché (évaporation, lyophilisation). Dans une variante de ce procédé, on utilise dans la première étape une solution méthanolique, éthanolique ou hydroalcoolique. L'extrait ainsi obtenu peut être évaporé à sec, le résidu étant utilisé tel quel ou après purification.
- Extrait enrichi en lactones sesquiterpéniques : Le matériel végétal est traité comme indiqué ci-dessus pour obtenir un extrait total hydroalcoolique qui est évaporé à sec. Cet extrait total est ensuite retraité par des solutions Hexane/acétate d'éthyle dans des proportions variables afin de concentrer les lactones sesquiterpéniques. Cette solution est filtrée et permet d'obtenir l'extrait enrichi en lactones sesquiterpéniques. Cet extrait peut également être séché par évaporation.
- Molécules pures : Les extraits, totaux ou enrichis en lactones sesquiterpéniques, peuvent être fractionnés par chromatographie afin de séparer les différentes lactones sesquiterpéniques, comme la vulgarine, la 4-épivulgarine ou encore la 2,3-dihydrovulgarine. Ces fractions permettent d'extraire des composés purs à partir de matériel végétal. Ces composés peuvent aussi être utilisés dans les préparations dermocosmétiques et pharmaceutiques pour les propriétés revendiquées précédemment. Des dérivés synthétiques et/ou hémisynthétiques peuvent également être revendiqués pour ces mêmes propriétés. De tels dérivés synthétiques et hémisynthétiques retrouvent tout leur intérêt dans le cas de maladies neurodégénératives où la molécule doit être optimisée afin de franchir certaines barrières naturelles.

Ces trois modes de préparation font partie intégrante de l'invention. Ces extraits et composés purs peuvent être utilisés tels quels sous forme liquide ou en poudre après séchage par atomisation, évaporation ou lyophilisation, ou encapsulée dans des liposomes ou autres vecteurs et supports pour une meilleure homogénéité de la composition et une meilleure diffusion du produit actif notamment sur la peau.

Comme indiqué précédemment, la Demanderesse a mis en évidence que les lactones sesquiterpéniques, et plus particulièrement la vulgarine, permettent de prévenir, de retarder ou de traiter les processus de mort cellulaire par apoptose en inhibant les caspases. En conséquence, les compositions de l'invention trouvent des applications dans les domaines pharmaceutiques, dermatologiques et cosmétiques, pour traiter et/ou prévenir des états pathologiques ou des troubles esthétiques résultant de la mort cellulaire par apoptose.

Les lactones sesquiterpéniques, plus particulièrement la vulgarine, et les extraits végétaux les contenant, peuvent être associés dans les compositions de l'invention avec d'autres composés présentant des propriétés diverses. Ainsi, il a été observé que l'activité anti-inflammatoire des lactones sesquiterpéniques ou des extraits végétaux les contenant peut encore être amélioré en association avec des substances augmentant la protection cutanée. A titre d'exemple mais de manière non limitative, on peut citer les associations avec des mucopolysaccharides, des vitamines, des céramides, des substances antiradicalaires, des filtres U.V.

De même, l'activité réparatrice des lactones sesquiterpéniques ou des extraits végétaux les contenant est particulièrement intéressante quand ils sont associés avec des substances ayant un effet cicatrisant comme des protéines, l'acide hyaluronique, des acides aminés, ou avec des substances anti-inflammatoires.

L'activité protectrice des lactones sesquiterpéniques ou des extraits les contenant vis-à-vis des radicaux libres et des UV est également très intéressante dans le domaine capillaire, notamment dans le cas d'association avec des substances facilitant le bon état du cuir chevelu et de celui du cheveu, comme des minéraux, des vitamines, des céramides, des extraits protéiques, des mucopolysaccharides, des acides de fleurs ou de fruits.

Ainsi, les compositions de l'invention sont tout particulièrement adaptées à une application topique pour prévenir et/ou traiter de nombreuses altérations cutanées résultant du processus de mort cellulaire par apoptose (Contet-Audonneau J.-L. et al, IFSCC, 2001, 4, 115-123).

L'invention a donc tout particulièrement pour objet l'utilisation cosmétique d'une lactone sesquiterpénique, et plus particulièrement de vulgarine, ou d'un extrait végétal les contenant, comme agent protecteur et réparateur de la peau et du système pileux comme les cheveux, notamment pour lutter contre les agressions externes liées à la pollution, au soleil, au stress oxydatif, au vieillissement, en contrecarrant les processus apoptotique et inflammatoire, conditionnés par les caspases.

Une application directe des compositions de l'invention concerne la lutte contre le vieillissement et la mort cellulaire induites par les UV. L'invention se rapporte aussi à l'une utilisation de ces compositions en tant que prolongateur de bronzage.

Pour une utilisation cosmétique, les compositions de l'invention peuvent se présenter sous la forme de crèmes, gels, lotions, laits, émulsions H/E et E/H, solutions, onguents, pulvérisateurs, huiles corporelles, lotions capillaires, shampooings, lotions après-rasage, savons, bâtons protecteurs des lèvres, bâtons et crayons pour maquillage.

Sous la forme de gel, elles comprennent des excipients appropriés tels que les esters de cellulose ou d'autres agents gélifiants, tels que le carbopol, la gomme guar, etc.

Ces compositions cosmétiques peuvent aussi prendre la forme de lotion ou solution dans laquelle les extraits et/ou molécules sont sous forme encapsulée, par exemple dans des microsphères. Ces microsphères peuvent par exemple être constituées de corps gras, d'agar et d'eau. Les agents actifs peuvent être aussi incorporés dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux. Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions cosmétiques de l'invention comprennent de l'ordre de 0,01 à 5% en poids, préférentiellement entre 0,1 et 2,5% d'agents actifs lorsqu'elles sont sous forme de poudre et de l'ordre de 0,01 à 25%, préférentiellement entre 0,5 et 10% lorsqu'elles sont sous forme encapsulée.

Pour la préparation de ces compositions, la lactone sesquiterpénique, et plus particulièrement la vulgarine, ou un extrait végétal, sont mélangés aux excipients généralement employés en cosmétique.

Les compositions cosmétiques de l'invention peuvent aussi contenir des additifs ou des adjuvants usuels en cosmétologie, comme par exemple des agents antibactériens ou des parfums mais aussi des lipides d'extraction et/ou de synthèse, polymères gélifiants et viscosants, tensio-actifs et émulsifiants, principes actifs hydro ou liposolubles, extraits de plantes, extraits tissulaires, extraits marins, actifs de synthèse.

L'utilisation cosmétique ou dermocosmétique d'extraits et/ou molécules comprend tous les soins du corps et de la peau y compris les produits solaires, protecteurs et bronzants, les produits anti-âge, anti-seborrhéïques, toniques, les produits assurant l'amélioration de l'aspect de la peau y compris le traitement acnéique, les rougeurs cutanées, le traitement du cuir chevelu et celui de la chute des cheveux.

Les compositions cosmétiques de la présente invention peuvent aussi comprendre d'autres principes actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, ainsi que d'autres principes actifs ayant une action sur la tonicité cutanée, la protection du cheveu.

Les compositions cosmétiques de la présente invention sont de préférence à utiliser quotidiennement en les appliquant une ou plusieurs fois par jour.

Les compositions cosmétiques de la présente invention sont très bien tolérées, elles ne présentent aucune phototoxicité et leur application sur la peau, pour des périodes de temps prolongées, n'implique aucun effet systémique.

L'invention concerne aussi l'utilisation d'une lactone sesquiterpénique choisie parmi la vulgarine la 4-épivulgarine, la 2,3 dihydrovulgarine ainsi que leurs sels et un ou plusieurs véhicules ou excipients adaptés à une application topique, ou un extrait végétal les contenant ou une composition les contenant pour la préparation de compositions destinées à traiter et/ou prévenir les troubles esthétiques résultant d'un processus de mort cellulaire par apoptose. Cette utilisation vise la préparation d'une composition destinées à inhiber une caspase, notamment la caspase 3. Ces compositions sont utiles pour prévenir et/ou traiter notamment des maladies dermatologiques liés à des activités séborrhéiques, acnéiques ou inflammatoires.

Ces compositions dermocosmétiques se présentent sous forme liquide, de poudre, de pâte ou d'émulsion, seule ou en combinaison avec d'autres substances. Elles comprennent de l'ordre de 0,01 à 25% en poids de lactone sesquiterpénique, et plus particulièrement de vulgarine, ou d'un extrait végétal les contenant.

Pour les préparations de ces compositions dermocosmétiques, les extraits et/ou lactones sesquiterpéniques issus des genres *Artemisia* et/ou *Achillea* sont mélangés à des excipients.

En conséquence, de manière générale, l'invention se rapporte à l'utilisation d'une lactone sesquiterpénique, plus particulièrement de vulgarine, ou d'un extrait végétal les contenant, pour la préparation d'une composition pharmaceutique, nutraceutique, dermatologique ou cosmétique destiné à prévenir et/ou traiter les pathologies et désordres résultant :
- de la sénescence des cellules de la peau et des articulations, comme dans le cas des pathologies articulaires liées au vieillissement et à la dégénérescence du cartilage, notamment dans le cas d'arthrose et de polyarthrite et autres affections inflammatoires du cartilage,
- d'une neurodégénérescence,
- d'états inflammatoires des systèmes cutanés, pileux et articulaires, induisant des démangeaisons, irritations, rougeurs chroniques, irritations et démangeaisons persistantes et des troubles fonctionnels de la fragilité capillaire
- d'un dysfonctionnement cellulaire du pancréas.

La portée des utilisations selon l'invention est telle que définie dans les revendications.

Une composition pharmaceutique selon l'invention comprend outre au moins un agent actif comme défini précédemment, un vecteur, ou un excipient pharmaceutiquement acceptable.

La synthèse et l'hémisynthèse de ces dérivés lactones sesquiterpéniques peuvent permettent d'augmenter les qualités biophysico-chimiques de tels composés afin de les rendre plus perméables aux différentes barrières biologiques de l'organisme. C'est le cas par exemple du passage de la barrière hémato-encéphalique dans le cas de revendications des propriétés de protection du système nerveux central. Toutes formulations cosmétiques intégrant de telles substances sont revendiquées pour les mêmes propriétés.

Dans le cadre d'une application nutraceutique, les compositions selon l'invention sont destinées à offrir une lactone sesquiterpénique, un dérivé ou analogue de celle-ci ou encore au moins un extrait végétal contenant au moins une lactone sesquiterpénique, avantageusement la vulgarine, à titre de nutriments pour les applications thérapeutiques et prophylactiques indiquées précédemment.

D'autres avantages et caractéristiques de l'invention apparaîtront des exemples qui suivent concernant la préparation d'agents actifs à base de lactones sesquiterpéniques, l'activité de la vulgarine dans des tests apoptotiques et sa toxicité. Il sera fait référence dans ces exemples aux dessins en annexe dans lesquels :

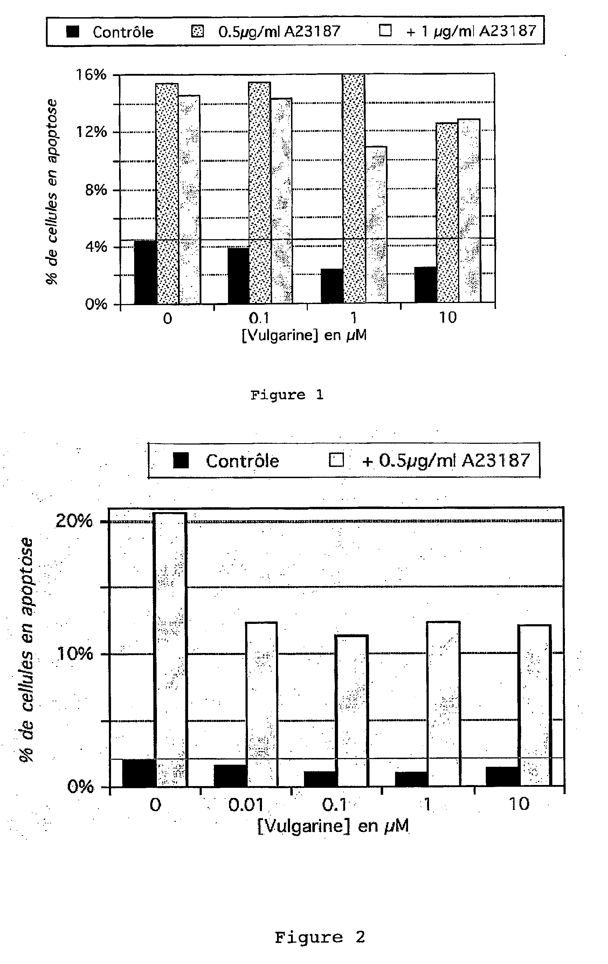

- la figure 1 représente le pourcentage de cellules JURKAT en apoptose après 12 heures d'incubation avec des quantités variables de vulgarine.
- la figure 2 représente le pourcentage de cellules JURKAT en apoptose après 24 heures d'incubation avec des quantités variables de vulgarine.

### Exemple 1 : Réalisation de l'extrait total, de l'extrait purifié et purification des principes actifs.

La partie aérienne d'*Artemisia* Sp ou d'*Achillea* Sp de la famille des Astéracées a été utilisée.

La partie aérienne a été séchée puis broyée en une poudre.

On a extrait la poudre avec un mélange eau/éthanol (5/95 en volume) à raison de 10 parties pour 1 partie de végétal, à température ambiante.

Après 5 heures d'agitation, on a filtré et/ou centrifugé, puis évaporé le filtrat et/ou mis le surnageant, à sec. Le rendement est bien entendu fonction de la plante traitée.

L'extrait total hydroalcoolique sec 1 ainsi obtenu peut être utilisé pour ses propriétés revendiquées. Un protocole analogue cité dans la littérature (Breton J.L. et al, Tetrahedron, 1985, 41, 3141) peut être appliqué.

Afin d'amplifier le rapport activité biologique/matière, il est possible de concentrer les molécules actives, c'est-à-dire les lactones sesquiterpéniques dans notre cas.

L'extrait total sec 1, précédemment obtenu, peut alors être repris dans un mélange de solvants hexane/acétate d'éthyle (1:4 v/v). Cet extrait 2 enrichi en lactones sesquiterpéniques peut alors être évaporé, séché et conservé sous atmosphère inerte (azote ou argon).

Une alternative serait d'extraire par ce même solvant hexane/acétate d'éthyle, les lactones sesquiterpéniques directement à partir des parties aériennes du végétal.

Cet extrait 2 enrichi en lactones sesquiterpéniques peut alors être utilisé pour ses propriétés revendiquées.

La purification des différentes lactones sesquiterpéniques peut être réalisée à partir des extraits 1 et/ou 2 par des mélanges hexane/acétate d'éthyle dans des proportions de polarité croissante (par exemple 4:1, 4:3, 1:1, 3:4, et 1:4 v/v). Les différentes fractions obtenues ont été chromatographiées avec un gradient hexane/acétate d'éthyle, puis regroupées selon leur profil chromatographique. Les lactones sesquiterpéniques pures obtenues sont fonction de la plante traitées. Ces différentes lactones pures peuvent être employées seules ou en associations qualitatives et/ou quantitatives entre elles, pour leurs propriétés biologiques revendiquées.

### Exemple 2 : Réalisation des tests apoptotiques 1 - Evaluation de l'activité de la vulgarine et de ses dérivés.

Les tests apoptotiques ont été réalisés sur des cellules de type Jurkat. La lignée cellulaire Jurkat (Clone E6-1, ATCC TIB 152), dérivée de cellule T humaine de leucémie aiguë, a été décrite dans la littérature (Weiss A. et al, Journal of Immunology, 1984, 133, 123). Les cellules ont été incubées dans un milieu de culture en présence 1 µL d'arséniate de sodium. La concentration finale en arséniate de sodium dans le milieu de culture est de 5 *µ*M. Afin de tester l'inhibition de l'apoptose induite par l'arséniate de sodium, les cellules ont été cultivées soit en absence (témoin) soit en présence de vulgarine 2,6 *µ*M ou d'un de ses dérivés.

L'apoptose a été quantifiée par cytometrie en flux. Les cellules sont rendues perméables et leurs noyaux sont teintés avec de l'iodure de propidium en présence de ribonucléase. Un minimum de 3000 cellules ont été analysées afin de quantifier l'apoptose cellulaire (Hotz M.A. et al, Cytometry, 1994, 15, 237).

Dans ces conditions expérimentales, le pourcentage d'inhibition de l'apoptose, en présence de 2,6 µM de vulgarine, est voisin de 100%. Il en est de même pour la 4-épivulgarine. Quant aux extraits, ils montrent des niveaux d'inhibition variables selon leur teneur en lactones sesquiterpéniques.

Ainsi, l'apoptose cellulaire est levée par les lactones sesquiterpéniques de manière dose dépendante. Ces lactones sesquiterpéniques ont donc un rôle protecteur au niveau cellulaire et moléculaire.

### Exemple 3 : Réalisation des tests apoptotiques 2 - Evaluation de l'activité de la vulgarine.

La lignée cellulaire humaine T Jurkat a été incubée avec l'ionophore A23187, inducteur d'apoptose, en présence ou absence de vulgarine en concentrations croissantes (de 0.001µM à 10 µM). Les populations cellulaires sont alors analysées par cytométrie en flux après marquage fluorescent (AnnexinV pour révéler les phosphatidylesérines en surface cellulaire = premier signes de l'apoptose - Iodure de Propidium pour révéler l'ADN des cellules nécrotiques) et différentiées en sous populations : cellules normales non marquées, cellules apoptotiques (marquage AnnexinV simple), cellules nécrotiques (marquage double AnnexinV et IP).

### Exemple 4 : Evaluation de la toxicité pour la vulgarine et ses dérivés.

Le test de viabilité cellulaire est décrit ci-après.

Les tests ont été réalisés sur des PBMCs (lymphocytes et monocytes) ou du sang total de donneurs sains humains. Tous les prélèvements ont été réalisés au Service d'Hépato-Gastroentérologie et d'Assistance Nutritive des Hôpitaux Universitaires de Strasbourg (Hôpital de Hautepierre), après approbation du protocole d'étude concernant les effets du célastrol par le CCPPRB n°1 d'Alsace.

Chez des volontaires sains, 20 ml de sang veineux périphérique sont prélevés le matin à jeun sur héparine. Le sang est ensuite dilué au demi dans de la solution saline équilibrée de Hank's dépourvue en Ca²⁺ et en Mg²⁺ (HBSS-CMF, Gibco BRL, Cergy, France) additionnée de 100 UI/ml de streptomycine (Biochrom KG, Berlin, Allemagne) et de 100 UI/ml de pénicilline (Biochrom KG). Les PBMCs (lymphocytes et monocytes) sont alors séparées par centrifugation sur Ficoll-Histopaque de densité 1,076 (Sigma, L'Isle d'Abeau-Chesnes, France) (30 min à 400 g) puis, après deux lavages dans du HBSS-CMF additionné d'antibiotiques (10 min à 250 g), resuspendues dans du milieu RPMI 1640 (Gibco BRL) additionné de 10 % (volume/volume) de sérum foetal de veau (SFV) inactivé à la chaleur (56°C, 30 min, Gibco BRL) et d'antibiotiques. Après comptage à la cellule de Malassez, les cellules sont incubées à raison de 2 x 10⁵ cellules/puits de 1 ml dans des plaques de 24 puits (Corning Inc., New York, USA) à 37°C dans un incubateur humidifié (air 95 % - CO₂ 5 %), en présence ou en absence des molécules étudiées préparées dans du DMSO (diméthyl sulfoxyde, Sigma ; maximum : 1 % (v/v) par puit), avec ou sans activation par du lipopolysaccharide de *Salmonella abortus equi* (LPS, 5 µg/ml) (Sigma) et de la phytohémagglutinine (PHA, 1 µg/ml) (Sigma) ajoutés à T₀. Après 24 heures d'incubation, les plaques sont centrifugées (15 min à 200 g), et les surnageants prélevés puis congelés à -80°C jusqu'au dosage.

La technique est basée sur le clivage par la déshydrogénase mitochondriale d'un sel de tétrazolium de couleur jaune en formazan de couleur orange. Les culots cellulaires (PBMCs et sang total) contenus dans les plaques de culture sont resuspendus dans du RPMI 1640 additionné d'hydroxide 2,3-*bis*-(2-méthoxy-4-nitro-5-sulfophényl)-5-[(phénylamino)carbonyl]-2*H*-tétrazolium (XTT, 1 mg/ml, Sigma), et de méthosulfate de phénazine (1,25 mM, Sigma), catalyseur de la réaction. Les plaques sont incubées 4 à 24 heures, puis lues en absorbance à 450 nm (filtre de référence : 620 nm). La viabilité cellulaire est estimée par rapport au témoin de cellules activées.

Les résultats obtenus sont rapportés dans tableau 1 ci-dessous.

**Tableau 1**

| | Pourcentage de viabilité cellulaire | | |
|---|---|---|---|
| Concentration (µM) | Vulgarine | 4-épivulgarine | 2,3-dihydrovulgarine |
| 30 | 102 | 100 | 105 |
| 10 | 104 | 103 | 108 |
| 1 | 119 | 99 | 105 |
| 0,1 | 102 | 103 | 104 |
| 0,01 | 112 | 105 | 110 |

Ces résultats montrent que ces dérivés de la vulgarine ne présentent aucun effet toxique de 0,01 µM à 30 µM.

## Revendications

1. Composition dermatologique, ou cosmétique **caractérisée en ce qu'**elle comprend comme agent actif au moins une lactone sesquiterpénique choisie parmi la vulgarine, la 4-épivulgarine, la 2,3 dihydrovulgarine ainsi que leurs sels et un ou plusieurs véhicules ou excipients adaptés à une application topique.

2. Composition selon la revendication 1 **caractérisée en ce que** lesdits sels sont formés avec des acides inorganiques choisis parmi le groupes des acides chlorhydrique, bromhydrique, sulfurique, phosphorique, nitrique ou équivalent, ou des sels formés avec des acides organiques tels que les acides acétique, oxalique, tartrique, succinique, malique, fumarique, maléique, ascorbique, benzoïque, tannique, alginique, polyglutamique, naphtalène sulfonique, naphtalène disulfonique et polygalacturonique.

3. Composition selon l'une quelconque des revendications 1 ou 2 **caractérisée en ce qu'**elle comprend au moins un extrait végétal contenant de la vulgarine, et/ou de la 4-épivulgarine, et/ou de la 2,3 dihydrovulgarine.

4. Composition selon l'une quelconque des revendications précédentes **caractérisée en ce que** ledit agent actif est obtenu par synthèse, hémisynthèse ou extraction à partir d'un extrait végétal.

5. Composition selon les revendications 3 ou 4 **caractérisée en ce que** l'extrait végétal est un extrait de plante de la famille des *Asteracae* et plus particulièrement un extrait *d'Artemisia Sp* ou d'*Achillea Sp.*

6. Composition selon l'une quelconque des revendications précédentes, **caractérisée en ce que** l'agent actif est utilisé à des concentrations variant entre 0,01 à 5 % en poids, préférentiellement entre 0,01 et 2,5 % en poids lorsque la composition est sous forme de poudre et de l'ordre de 0,01 à 25 %, préférentiellement entre 0,5 et 10 % lorsque la composition est sous forme encapsulée.

7. Composition selon l'une quelconque des revendications précédentes, **caractérisée en ce qu'**elle comprend en outre au moins un actif complémentaire choisi parmi le groupe des substances augmentant la protection cutanée telles que les mucopolysaccharides, les vitamines, les céramides, les substances antiradicalaires, les filtres U.V, le groupe des substances facilitant le bon état de cuir chevelu et celui du cheveux telle que les minéraux, les vitamines, les céramides, les extraits protéiques, les mucopolysaccharides, les acides de fleurs ou de fruits, le groupe des principes actifs complémentaires tels que les protecteurs solaires, les antirides, les anti radicalaires et antioxydants, les anti-irritants, les principes actifs ayant une action sur la nutrition cellulaire, la respiration cellulaire, les traitements anti-séborrhéiques, sur la tonicité cutanée, sur la protection du cheveu ; le groupe des substances ayant un effet cicatrisant telles que les protéines, l'acide hyaluronique, les acides aminés ou le groupe des substances anti-inflammatoires.

8. Compositions selon l'une quelconque des revendications précédentes **caractérisées en ce qu'**elle comprend en outre des additifs ou des adjuvants usuels en cosmétique tels que des agents antibactériens, des parfums, des lipides d'extraction et/ou de synthèse, polymère gélifiants et viscosants, tensio-actifs et émulsifiants, principes actifs hydro ou liposolubles, extraits de plantes, extraits tissulaires, extraits marins, actifs de synthèse.

9. Composition selon l'une quelconque des revendications précédentes, **caractérisée en ce qu'**elle peut prendre la forme de lotion ou solution dans laquelle les extraits et/ou les molécules sont encapsulés sous la forme de microsphère, sont incorporés dans des vecteurs de type liposomes, glycosphères, dans des chylomicrons, des macro-, micro- ou nano-particules ainsi que des macro-, micro- ou nano-capsules, adsorbés sur des polymères organiques poudreux, les talcs, bentonites et autres supports minéraux.

10. Composition selon l'une quelconque des revendications précédentes, **caractérisée en ce qu'**elle peut se présenter sous la forme de crèmes, gels, lotions, laits, émulsions H/E et E/H, solutions, onguents, pulvérisateurs, huiles corporelles, lotions capillaires, shampoings, lotions après-rasage, savons, bâtons protecteurs des lèvres, bâtons et crayons pour maquillage.

11. Utilisation de la composition selon l'une quelconque des revendications 1 à 10 pour la préparation d'une composition destinée à traiter et/ou prévenir les troubles esthétiques résultant d'un processus de mort cellulaire par apoptose.

12. Utilisation selon la revendication 11 pour la préparation d'une composition destinée à inhiber une caspase, notamment la caspase 3.

13. Utilisation de la composition selon l'une quelconque des revendications 1 à 10 pour la préparation d'une composition destinée à protéger et réparer la peau et le système pileux, notamment lutter contre le vieillissement et les agressions externes liées à la pollution, au soleil, au stress oxydatif et/ou lutter contre le vieillissement et la mort cellulaire induite par les UV.

14. Utilisation de la composition selon l'une quelconque des revendications 1 à 10 pour la préparation d'une composition destinée à la préparation des soins du corps et de la peau tels que les produits solaires, protecteurs et bronzants, les produits anti-âge, anti-séborrhéiques, toniques, les produits assurant l'amélioration de l'aspect de la peau y compris le traitement acnéique, les rougeurs cutanées, le traitement du cuir chevelu et celui de la chute des cheveux, les produits prolongateurs de bronzage.

15. Utilisation de la composition selon l'une quelconque des revendications 1 à 10 pour la préparation d'une composition destinée à une activité anti-inflammatoire et/ou dermoprotectrice.

16. Utilisation d'au moins une lactone sesquiterpénique choisie parmi la vulgarine, la 4-épiwlgarine, la 2,3 dihydrovulgarine pour la fabrication d'un médicament destiné à prévenir et/ou traiter les maladies dermatologiques liées à des activités séborrhéiques, acnéiques ou inflammatoires, et/ou à traiter et/ou prévenir les états pathologiques ou les troubles résultant d'un processus de mort cellulaire par apoptose, ledit médicament étant administré par voie topique ou nutraceutique.

## Claims

1. Dermatological or cosmetic composition, **characterised in that** is comprise, as an active agent, it includes at least one sesquiterpene lactone chosen from among vulgarin, 4-epivulgarin, 2,3-dihydrovulgarin as well as the salts thereof and one or more vehicles or excipients suitable for topical application.

2. Composition of claim 1, **characterised in that** said salts are formed with inorganic acids chosen from the hydrochloric, hydrobromic, sulphuric, phosphoric, nitric or equivalent acid groups, or salts formed from organic acids such as acetic, oxalic, tartaric, succinic, malic, fumaric, maleic, ascorbic, benzoic, tannic, alginic, polyglutamic, naphthalene sulphonic, naphthalene disulfonic and polygalacturonic acid.

3. Composition according any of claims 1 or 2, **characterised in that** it comprises at least one vegetable extract containing vulgarin, and/or 4-epivulgarin, and/or 2,3-dihydrovulgarin.

4. Composition according any of the preceding claims, **characterised in that** said active agent is obtained from synthesis, hemisynthesis or extraction from vegetable extract.

5. Composition according claims 3 or 4, **characterised in that** the vegetable extract is a plant extract of the Asteracae family and, more particularly, an extract of *Artemisia Sp* or *Achillea Sp.*

6. Composition according any of the preceding claims, **characterised in that** the active agent is used at concentrations varying between 0.01 to 5% by weight, preferably between 0.01 and 2.5% by weight, when the composition is in powdered form, and of the order of 0.01 to 25% by weight, preferably between 0.5 and 10%, when the composition is in capsulated form.

7. Composition according any of the preceding claims, **characterised in that** it further comprises at least one additional active agent chosen from among the group of substances increasing skin protection, such as mucopolysaccharides, vitamins, anti-radical substances, UV filters, the group of substances contributing to healthy scalp and hair, such as minerals, vitamins, ceramides, protein extracts, mucopolysaccharides, flower and fruit acids, the group of additional active principles such as sunscreens, anti-wrinkle agents, anti-radicals and anti-oxidants, active principles having an affect on cell nutrition, cellular respiration, anti-seborrheic treatments, on skin tone, hair protection ; the group of substances having a healing affect, such as proteins, hyaluronic acid, amino acids or the group of anti-inflammatory substances.

8. Composition according any of the preceding claims, **characterised in that** it further comprises conventional cosmetic additives or adjuvants such as anti-bacterial agents, fragrances, extraction and/or synthesis lipids, gel-forming and thickening polymers, surfactants and emulsifiers, water- or lipid-soluble active principles, plant extracts, tissue extracts, sea extracts and synthesis active principles.

9. Composition according any of the preceding claims, **characterised in that** it can be in the form of a lotion or solution in which the extracts and/or molecules are encapsulated in the form of a microsphere, incorporated into liposome-type carriers, glycospheres, into chylomicrons, macro-, micro- or nano-particles as well as macro-, micro or nano-capsules, absorbed on powdery organic polymers, talcs, bentonites and other mineral supports.

10. Composition according any of the preceding claims, **characterised in that** it can be in the form of creams, gels, lotions, milks, O/W and W/O emulsions, solutions, ointments, sprays, body oils, hair lotions, shampoos, after-shave lotions, soaps, lip balms, and make-up sticks and pencils.

11. Use of the composition as claimed in any of claims 1 to 10 for the preparation of a cosmetic intended to treat and/or prevent appearance-related problems resulting from an apoptotic cell death process.

12. Use of claim 11 for the preparation of a composition intended to inhibit a caspase, in particular Capsase 3.

13. Use of the composition according any of claims 1 to 10 for the preparation of a composition intended to protect and repair the skin and body hair, in particular to combat ageing and external stress related to pollution, the sun and oxidative stress and/or to combat UV-induced ageing and cell death.

14. Use of the composition according any of claims 1 to 10 for preparing a composition intended for the preparation of body and skin care products such as sun-screening and self-tanning sun products, anti-ageing, anti-seborrheic, toning products, products providing improved skin appearance, including treatment of acne, skin rashes, treatment of the scalp and that of hair loss, and tan-extending products.

15. Use of the composition as claimed in any of claims 1 to 10 for the preparation of a composition intended to have an anti-inflammatory and/or dermoprotective activity.

16. Use of at least one sesquiterpene lactone chosen from among vulgarin, 4-epivulgarin and 2,3-dihydrovulgarin for the manufacture of a drug intended to prevent and/or treat skin diseases associated with seborrheic, acne-related or inflammatory activities, and/or to treat and/or prevent disease conditions or disorders resulting from an apoptotic cell death process, said drug being administered topically or in the form of a nutraceutical.

## Patentansprüche

1. Dermatologische oder kosmetische Zusammensetzung, **dadurch gekennzeichnet, dass** sie als Wirkstoff mindestens ein Sesquiterpen-Lakton umfasst, ausgewählt unter Vulgarin, 4-Epivulgarin, 2,3-Dihydrovularin sowie ihren Salzen und einem oder mehreren Vehicula oder Hilfsstoffen, die an eine topische Anwendung angepasst sind.

2. Zusammensetzung nach Anspruch 1, **dadurch gekennzeichnet, dass** die Salze mit anorganischen Säuren gebildet sind, die unter den Gruppen der Chlorwasserstoff-, Bromwasserstoff-, Schwefel-, Phosphor-, Salpeter- oder ähnlichen Säuren oder Salzen gewählt werden, die mit organischen Säuren wie z.B. der Essig-, Oxal-, Wein-, Bernstein, Apfel-, Fumar-, Malein-, Ascorbin-, Benzoe-, Gerb-, Algin-, Polyglutamin-, Naphtalen-Sulfon-, Naphtalen-Disulfon- und Polygalakturonsäure gebildet sind.

3. Zusammensetzung nach einem der Ansprüche 1 oder 2, **dadurch gekennzeichnet, dass** sie mindestens einen pflanzlichen Extrakt umfasst, der Vulgarin bwz. 4-Epivulgarin bwz. 2,3-Dihydrovulgarin enthält.

4. Zusammensetzung nach einem der vorhergehenden Ansprüche, **dadurch gekennzeichnet, dass** der Wirkstoff durch Synthese, Hemisynthese oder Extraktion aus einem pflanzlichen Extrakt erhalten wird.

5. Zusammensetzung nach Anspruch 3 oder 4, **dadurch gekennzeichnet, dass** der pflanzliche Extrakt ein Pflanzenextrakt der Familie Asteracae und insbesondere ein Extrakt von *Artemisia Sp.* oder *Achillea Sp.* ist.

6. Zusammensetzung nach einem der vorhergehenden Ansprüche, **dadurch gekennzeichnet, dass** der Wirkstoff in Konzentrationen verwendet wird, die zwischen 0,01 und 5 Gew.-%, insbesondere zwischen 0,01 und 2,5 Gew.-% variieren, wenn die Zusammensetzung in Form von Pulver vorliegt, und zwischen 0,01 und 25 Gew.-%, insbesondere zwischen 0,5 und 10 Gew.-%, wenn die Zusammensetzung in Form von Kapseln vorliegt.

7. Zusammensetzung nach einem der vorhergehenden Ansprüche, **dadurch gekennzeichnet, dass** sie außerdem mindestens einen zusätzlichen Wirkstoff umfasst, der unter der Gruppe der Substanzen gewählt wird, die den Schutz der Haut erhöhen, wie z.B. die Mucopolysaccaride, die Vitamine, die Ceramide, die antiradikalen Substanzen, die U.V.-Filter, die Gruppe der Substanzen, die den guten Zustand der Kopfhaut und der Haare ermöglichen, wie z.B. die Mineralien, die Vitamine, die Ceramide, die Proteinextrakte, die Mucopolysaccaride, die Säuren von Blumen oder Früchten, die Gruppe der zusätzlichen Wirkstoffe wie z.B. die Sonnenschutzmittel, die Mittel gegen die Faltenbildung, die Antiradikale und Antioxidationsmittel, die Anti-Reizmittel, die Wirkstoffe mit einer Wirkung auf die Zellernährung, die Zellatmung, die antiseborrhöischen Behandlungen, auf die Tonizität der Haut, auf den Schutz der Haare; wobei die Gruppe der Substanzen eine narbenbildende Wirkung hat, so z.B. die Proteine, die Hyaluronsäure, die Aminosäuren oder die Gruppe der antiinflammatorischen Substanzen.

8. Zusammensetzung nach einem der vorhergehenden Ansprüche, **dadurch gekennzeichnet, dass** sie außerdem Zusatzstoffe oder Hilfsstoffe umfasst, die in der Kosmetik üblich sind, wie z.B. antibakterielle Mittel, Parfums, Extraktions- bwz. Syntheselipide, Gelier- und Viskosemittel, Tenside und Emulgatoren, wasser- oder fettlösliche Wirkstoffe, Pflanzenextrakte, Gewebeextrakte, Meeresextrakte, synthetische Wirkstoffe.

9. Zusammensetzung nach einem der vorhergehenden Ansprüche, **dadurch gekennzeichnet, dass** sie die Form einer Lotion oder Lösung annehmen kann, in der die Extrakte bwz. Moleküle in der Form einer Mikrosphäre eingekapselt werden, in Vektoren des Typs Liposomen, Glycosphären, in Chylomikronen, Makro-, Mikro- oder Nanopartikel ebenso wie Makro-, Mikro oder Nanokapseln eingebaut werden, absorbiert auf pulvrigen organischen Polymeren, Talkmineralien, Bentoniten und anderen mineralischen Untergründen.

10. Zusammensetzung nach einem der vorhergehenden Ansprüche, **dadurch gekennzeichnet, dass** sie in Form von Cremes, Gels, Lotionen, Milch, H/E- und E/H-Emulsionen, Lösungen, Salben, Zerstäubern, Körperölen, Haarlotionen, Shampoos, After-Shave-Lotionen, Seifen, Lippenstiften, Betoniten und Make-up-Stiften vorkommen können.

11. Verwendung der Zusammensetzung nach einem der Ansprüche 1 bis 10 für die Zubreitung einer Zusammensetzung, die für die Behandlung bwz. die Vorbeugung der ästhetischen Störungen bestimmt ist, die sich aus dem Prozess des Zelltodes durch Apoptose ergeben.

12. Verwendung nach Anspruch 11 für die Zubereitung einer Zusammensetzung zur Hemmung einer Caspase, insbesondere der Caspase 3.

13. Verwendung der Zusammensetzung nach einem der Ansprüche 1 bis 10 für die Zubereitung einer Zusammensetzung zum Schutz und zur Wiederherstellung der Haut und der Behaarung, insbesondere zum Kampf gegen die Alterung und die externen Aggressionen im Zusammenhang mit der Umweltverschmutzung, der Sonne, dem oxidativen Stress bzw. zum Kampf gegen die Alterung und den Zelltod durch UV-Strahlen.

14. Verwendung der Zusammensetzung nach einem der Ansprüche 1 bis 10 für die Zubereitung einer Zusammensetzung zur Zubereitung von Körper- und Hautpflegemitteln wie z.B. Sonnenprodukten, Schutz- und Bräunungsmitteln, Anti-Aging-Produkten, antiseborrhöischen Mitteln, Stärkungsmitteln, Produkten, die die Verbesserung des Aussehens der Haut sicherstellen, darin eingeschlossen die Behandlung von Akne, Hautrötungen, die Behandlung der Kopfhaut sowie die Behandlung von Haarausfall, Produkten zur Verlängerung der Bräunung.

15. Verwendung der Zusammensetzung nach einem der Ansprüche 1 bis 10 für die Zubreitung einer Zusammensetzung zur antiinflammatorischen bzw. hautschützenden Aktivität.

16. Verwendung von mindestens einem Sesquiterpen-Lakton, ausgewählt unter Vulgarin, 4-Epivulgarin, 2,3-Dihydrovularin für die Herstellung eines Medikamentes zur Vorbeugung bwz. zur Behandlung der dermatologischen Krankheiten, die mit seborrhöischen Akne- oder inflammatorischen Aktivitäten verbunden sind, bwz. zur Behandlung bwz. zur Vorbeugung der pathologischen Zustände oder der Störungen, die sich aus einem Prozess des Zelltodes durch Apoptose ergeben, wobei das Medikament auf topischem oder nutrazeutischem Weg verabreicht wird.
